# EUROPEAN PATENT APPLICATION

(11) **EP 2 036 535 A1**
(43) Date of publication of application: **18.03.2009**
(21) Application number: 08163731.6
(22) Date of filing: 05.09.2008
(51) Int. Cl.: A61K 8/31, A61K 8/67, A61K 8/92, A61Q 19/08

(54) **Vitamin containing cream**

(30) Priority: 07.09.2007 DK 200701281
(71) Applicant: Beauté Pacifique ApS, 9560 Hadsund (DK)
(72) Inventor: Christensen, Flemming Kjærgaard, 9560, Hadsund (DK)
(74) Representative: Gregersen, Niels Henrik

(57) **Abstract**

A vitamin-containing cream for application on the skin of the face and of the body, containing glycerol, phenoxyethanol/ethyhexylglycerol, tetrasodium EDTA, lemon, aloe vera, ascorbic acid, polysorbate-20, glyceryl stearate (PEG-100 stearate), isopropyl palmitate, cetyl ester, dimethicone, squalane, caprylic/capic triglycerol, lanolin alcohol, oleyl alcohol, tocopherol, tocopherol acetate, retinyl palmitate, retinyl acetate, retinyl propionate, unsaponifiable avocado oil and water. In a simple way is hereby achieved a cream which, as a supplement to the natural sun-induced vitamin D of the skin, can supply the skin with extra vitamin D. The cream furthermore contains types of vitamin A esters, each providing the whole skin a significant structural improvement by age changes as well as actinic degradation.

## Description

### Field of the Invention

The present invention concerns a vitamin-containing cream for application on the skin of the face and the body and of the kind indicated in the preamble of claim 1.

### Background of the Invention

Many people are very occupied with avoiding the action of sunshine because of the focus on the fact that too much sun may cause skin cancer. Vitamin D is only formed in the skin, and the most recent research has concluded that a deficiency of vitamin D may lead very serious health problems.

### Object of the Invention

The invention has the object of indicating a new and improved vitamin-containing cream of the kind mentioned in the introduction, and which further has appeared to have the very significant effect that the skin is supplied with extra vitamin D as a supplement to the vitamin D naturally induced by the sun in the skin.

### Description of the invention

The vitamin-containing cream according to the invention is characterised in that in a high-pressure homogenised cream it contains glycerol, phenoxyethanol/ethylhexylglycerol, tetrasodium EDTA, lemon, aloe vera, ascorbic acid, polysorbate-20, glyceryl stearate (PEG-100 stearate), isopropyl palmitate, cetyl ester, dimethicone, squalane, caprylic/capic triglycerol, lanolin alcohol, oleyl alcohol, tocopherol, tocopherol acetate, retinyl palmitate, retinyl acetate, retinyl propionate, unsaponifiable avocado oil and water.

In a simple way is hereby achieved a cream which as supplement to the natural suninduced vitamin D of the skin can supply the skin with extra vitamin D. The cream furthermore contains types of vitamin A esters each providing the whole skin a significant structural improvement by age changes as well as actinic degradation. The cream moreover contains two types of vitamin E acting as antioxidants. The cream has a very strong effect as an anti-age-skin care agent for the entire body. The cream uses the patented penetration system, cf. DK/EP 1353645 T3, which leads the active substances deep into the skin.

### Detailed Description of the Invention

Persea Gratissima Oil or **avocado oil** is rich in lanolin acid which the skin is lacking with increasing age, and furthermore avocado oil contains a number of other vitamins that penetrate into the skin together with the oil. Unsaponifiable avocado oil constitutes a microscopic part of the normal avocado oil but is here used in pure form. In an entirely unique and natural way, the D-FORCE products are provided vitamin D which thus may be supplied to the skin by means of the above mentioned patented penetration system (delivery system squalane).

**Squalane** is an oil occurring naturally in the skin and constituting an important part of the lipid system. Young skin already contains an optimal amount of squalane, but with age the level drops drastically. The sun- and age-damaged skin therefore absorbs squalane particularly well, and the penetration ability is unusually great. Nanometric drops of squalane functions as efficient carriers of vitamin As and other oil soluble active ingredients. Lack of squalane in the skin causes too premature ageing, such as dryness and lack of elasticity. Areas that are dry and changed by age are e.g. hands, elbows, heels etc.; they become surprising quickly soft when squalane is applied with the right formulation.

**Tocopheryl acetate** is a type of vitamin E playing an important role in protecting the skin as well as the cream against damaging free radicals.

**Rectinyl palmitate** is a particularly stable type of vitamin A which we have formulated into the cream such that it remains very active, both under storage and when applied to the skin. Encapsulated in nanometric droplets of squalane, vitamin A can work deep down into the skin in amounts which are sufficient to stimulate the skin cells to restore lost collagen fibre structure. Wrinkles and fine lines are thereby reduced, and the structure is substantially rejuvenated in the entire thickness of the skin. In the above mentioned patented combination with squalane operating as an efficient carrier through the skin, vitamin A has a quite unique and very efficient anti-age effect. Ultra-studies have shown anti-age effects corresponding to 10-20 years' rejuvenation.

**Tocopherol** is a source of vitamin E functioning as a natural moisturiser which is also able toe reduce fine lines and wrinkles in the skin.

**Rectinyl acetate** is, as **Rectinyl palmitate,** a particularly stable type of vitamin A which as a strong effect on the skin. Just as rectinyl palmitate, rectinyl acetate provides that the skin cells will be able to restore and repair age and sun damages. When both types of vitamin A are used, the action is further enhanced.

**Rectinyl propionate** is a third, very stable vitamin A having the hitherto strongest anti-age effect in the market, and it stabilises other types of vitamin A such that the total effect is considerably increased.

The cream according to the invention may be used for whole-body anti-age treatment of the skin, in particular where efficient protection against age changes and sun damage is desired, and where the skin does not receive enough sunshine on a daily basis for the formation of vitamin D in the skin to the desired extent. The cream is applied morning and evening according to need and directions. The cream is applied in a very thin layer and is massaged into the skin in an amount which is not felt greasy, corresponding to good penetration.

The cream according to the invention represents a high-tech breakthrough within skin care. Now it is possible to supply the skin with entirely natural vitamin D which the skin frequently does not form by itself, because most people are now focused on the fact that too much sun may lead to skin cancer. Vitamin D is only formed in the skin, and recent research concludes that a deficit of vitamin D in the skin may cause very serious health problems.

Vitamin D through dietary supplements is very limited by legislation, and experts differ on the risks connected with oral ingestion. The cream may be used as anti-age cream in the morning and applied so thinly that the cream is penetrating completely into the skin in the course of few minutes. After that, an oil-free lotion is e.g. applied as the daily moisturiser.

### Example 1

| Cream containing: | % |
|---|---|
| Water | 44.39 |
| Glycerol (humectant) | 7 |
| Phenoxyethanol/Ethylhexyl glycerol (preservation) | 1 |
| Tetrasodium EDTA (chelating agent) | 0.1 |
| Lemon (extract) | 7 |
| Aloe Vera (extract) | 3.85 |
| Ascorbic acid 10 % (anti-oxidant) | 0.04 |
| Polysorbate-20 (emulgator) | 8 |
| Lanolin (humectant) | 2.5 |
| Unsaponifiable avocado oil | 10 |
| Glyceryl Stearate (PEG-100) (thickener) | 3 |
| Isopropyl Palmitate (emmolient) | 1.1 |
| Cetyl Esters (emmolient, texture provider) | 2 |
| Dimethicone (emmolient) | 0.9 |
| Squalane (penetration enhancer) | 4.82 |
| Caprylic/Capic Triglycerol (penetration enhancer) | 1.4 |
| Lanolin Alcohol, Oleyl Alcohol (moisture) | 1.5 |
| Tocopherol (anti-oxidant) | 0.5 |
| Tocopherylacetate (anti-oxidant) | 1 |
| Retinyl palmitate | 1 |
| Retinyl acetate | 0.6 |
| Retinyl propionate | 0.3 |

### Example 2

| Cream containing: | % |
|---|---|
| Water | 35.39 |
| Glycerol (humectant) | 7 |
| Phenoxyethanol/Ethylhexylglycerol (preservation) | 1 |
| Tetrasodium EDTA (chelating agent) | 0.1 |
| Lemon (extract) | 7 |
| Aloe Vera (extract) | 3.85 |
| Ascorbic acid 10 % (anti-oxidant) | 0.04 |
| Polysorbate-20 (emulgator) | 8 |
| Lanolin (humectant) | 2.5 |
| Unsaponifiable avocado oil | 17 |
| Glyceryl Stearate (PEG-100) (fortykker) | 3 |
| Isopropyl Palmitat (emmolient) | 1.1 |
| Cetyl Esters (emmolient, texture provider) | 2 |
| Dimethicon (emmolient) | 0.9 |

| Squalane (penetration enhancer) | 4.82 |
|---|---|
| Caprylic/Capic Triglycerol (penetration enhancer) | 1.4 |
| Lanolin Alcohol, Oleyl Alcohol (moisture) | 1.5 |
| Tocopherol (anti-oxidant) | 0.5 |
| Tocopheryl acetate (anti-oxidant) | 1 |
| Retinyl palmitate | 1 |
| Retinyl acetate | 0.6 |
| Retinyl propionate | 0.3 |

## Claims

1. A vitamin-containing cream for application on the skin of the face and of the body, containing glycerol, phenoxyethanol/ethyhexylglycerol, tetrasodium EDTA, lemon, aloe vera, ascorbic acid, polysorbate-20, glyceryl stearate (PEG-100 stearate), isopropyl palmitate, cetyl ester, dimethicone, squalane, caprylic/capic triglycerol, lanolin alcohol, oleyl alcohol, tocopherol, tocopherol acetate, retinyl palmitate, retinyl acetate, retinyl propionate, unsaponifiable avocado oil and water.

2. Vitamin-containing cream according to claim 1, containing the following percentages:
| Water | 44.39 |
|---|---|
| Glycerol | 7 |
| Phenoxyethanol/Ethyl hexyl glycerol | 1 |
| Tetrasodium EDTA | 0.1 |
| Lemon | 7 |
| Aloe Vera | 3.85 |
| Ascorbic acid 10 % | 0.04 |
| Polysorbate-20 | 8 |
| Lanolin | 2.5 |
| Unsaponifiable avocado oil | 10 |
| Glyceryl Stearate (PEG-100) | 3 |
| Isopropyl Palmitate | 1.1 |
| Cetyl Esters | 2 |
| Dimethicone | 0.9 |
| Squalane | 4.82 |
| Caprylic/Capic Triglycerol | 1.4 |
| Lanolin Alcohol, Oleyl Alcohol | 1.5 |
| Tocopherol | 0.5 |
| Tocopheryl acetate | 1 |
| Retinyl palmitate | 1 |
| Retinyl acetate | 0.6 |
| Retinyl propionate | 0.3 |

3. Vitamin-containing cream according to claim 1, preferably containing the following percentages:
| | |
|---|---|
| Water | 35.39 |
| Glycerol | 7 |
| Phenoxy ethanol/Ethylhexylglycerol | 1 |
| Tetrasodium EDTA | 0.1 |
| Lemon | 7 |
| Aloe Vera | 3.85 |
| Ascorbic acid 10 % | 0.04 |
| Polysorbate-20 | 8 |
| Lanolin | 2.5 |
| Unsaponifiable avocado oil | 17 |
| Glyceryl Stearate (PEG-100) | 3 |
| Isopropyl Palmitate | 1.1 |
| Cetyl Esters | 2 |
| Dimethicone | 0.9 |
| Squalane | 4.82 |
| Caprylic/Capic Triglycerol | 1.4 |
| Lanolin Alcohol, Oleyl Alcohol | 1.5 |
| Tocopherol | 0.5 |
| Tocopheryl acetate | 1 |
| Retinyl palmitate | 1 |
| Retinyl acetate | 0.6 |
| Retinyl propionate | 0.3 |
